(19)

Europäisches
Patentamt
European
Patent Office
Office européen
des brevets

(11) **EP 1 744 261 A1**

(12) **EUROPEAN PATENT APPLICATION**

(43) Date of publication:
**17.01.2007 Bulletin 2007/03**

(51) Int Cl.:
*G06F 19/00* (2006.01)

(21) Application number: **05015085.3**

(22) Date of filing: **12.07.2005**

(84) Designated Contracting States:
**AT BE BG CH CY CZ DE DK EE ES FI FR GB GR
HU IE IS IT LI LT LU LV MC NL PL PT RO SE SI
SK TR**
Designated Extension States:
**AL BA HR MK YU**

(71) Applicant: **Medizinische Universität Wien
1090 Vienna (AT)**

(72) Inventors:
• **Thurner, Stefan Dr. Mag.
1080 Wien (AT)**

• **Wick, Nicholaus Dr.
1190 Wien (AT)**
• **Biely, Christoly DI
3411 Weidling (AT)**
• **Stokic, Dejan Dipl.-Phys.
1090 Wien (AT)**

(74) Representative: **Vossius & Partner
Siebertstrasse 4
81675 München (DE)**

(54) **Method for calculation of statistical characteristics for a set of gene expression values**

(57)     The invention is concerned with the field of analysis of data resulting from gene profiling experiments, and provides a method for calculation of statistical characteristics e.g. standard deviation values for a set of value pairs $(X_n|Y_n)$, comprising the steps of a) grouping pairs and b) calculating the characteristics e.g. standard deviation for each group.

Fig. 5

group genes of similar expression profile into slices (e.g. by means of rotation and subsequent taking equally populated groups)

compute statistics within slices

display resulting relative statistics in the original plot (e.g. colour coded for different statistical significance regions)

**Description**

**Field of the invention**

The invention is concerned with the field of analysis of data resulting from gene profiling experiments.

**Background of the invention**

In gene expression profiling, large numbers of probes are commonly hybridized by RNA extracted from cells or tissue to be analyzed. The method allows the parallel determination of expression levels measured in terms of RNA abundance of a multitude of genes. An inherent problem of the method is a certain variation of the detected expression levels. This variation is caused by the measurement method or by factors inherent to the analyzed sample. This constitutes a problem, because in analyzing the samples, one must determine which changes in expression level (e.g. upon application of a stimulus) are significant and which merely reflect statistical variation.

It is also important to detect those genes which are very unlikely to belong to statistical noise but constitute biological relevance. The proposed method of the present invention is especially designed to single out these genes in a statistically consistent and homogenous way over a large range of expression levels.

Commonly, results of gene profiling experiments are often displayed in an X-Y scatter graph. The X axis represents the expression level in condition 1 (e.g., control), whereas the Y axis represents the expression level in condition 2 (e.g., induced with a stimulus). The X and Y axis may also represent different cell lines or tissues (e.g., diseased versus healthy tissue) that are to be compared.

Each gene is represented on the plot by a single dot. Genes that are expressed equally in both conditions or tissues will be located on a straight line crossing the intersection of both axes and having a 45° angle. Dots above the line correspond to genes that are more expressed in the condition reflected by the Y axis, whereas dots below the line correspond to genes that are more expressed in the condition reflected by the X axis.

In the state of the art of the analysis of gene profiling data, thresholds are used to determine which genes might be over-expressed at relevant levels. For instance, one may set the threshold at a factor of 2 or more so that all genes whose expression level are less than 2 fold compared to the other are considered insignificant changes. Alternatively, also standard t-tests and variants of it are in use.

The 2-fold (or x-fold) deviation is often used because at these levels experimental checks as e.g. real-time PCR work reasonably well.

The 2-fold (or x-fold) deviation can be marked directly in that graph (often in double logarithmic form) as two lines in parallel above and below the said line. Dots located inside of the area delineated by these two lines will be below the 2-fold (x-fold) deviation threshold and will be considered genes that have not significantly changed.

The inventors state that the definition of the x-fold deviation is highly critical to the identification of genes associated with the different conditions. For instance, when a cancerous tissue and a healthy tissue of the same type are compared, many changes in expression levels are detected. The physiologically most important of these may not be the strongest change in expression level (e.g., a more than 30-fold change), but may very well be a minor change in a gene that drives or reflects the most important physiological changes. At the same time, a statistically insignificant change would merely reflect clutter and have no informational value and thus not merit further time-consuming investigation.

**Summary of the invention**

The object of the present invention is to provide an improved method of thresholding in a method for calculation of statistical characteristics for a set of gene expression values. This object is achieved with the features of the claims.

Generally, the present invention relates to methods of calculating distribution functions and their characterization, such as e.g. their standard deviation for a set of gene expression values. More specifically, the invention provides a method for calculation of distributions and their characteristics such as e.g. their standard deviation values for a set of gene expression value pairs $(X_n \mid Y_n)$, comprising the steps of a) grouping pairs and b) calculating the distribution characteristics such as the standard deviation for each group.

In a preferred method of determining the significance level of changes in gene expression patterns according to the present invention, the gene expression values $(X_n \mid Y_n)$ are first grouped according to their expression level. This is preferably done by rotating the expression levels displayed as dots on a X-Y-graph as described above by 45°. Each gene i, i.e. pair $(X_i, Y_i)$ will be rotated by applying the rotation matrix M on that pair: $(X_i', Y_i')=M(X_i, Y_i)$, where M is a 2x2 matrix whose components are given by $M_{11}=\cos(\varphi)$, $M_{12}=\sin(\varphi)$, $M_{21}=-\sin(\varphi)$ and $M_{22}=-\cos(\varphi)$, where $\varphi$ is the desired rotation angle of -45°. The line on which all unchanged genes are displayed is now falling on the Y axis. Alternatively, a clockwise rotation is performed by setting $\varphi = +45°$.

After rotation, the space around the Y axis is preferably divided by lines parallel to the X axis. More preferably, these lines are equally spaced. As an alternative, it is preferred to use a variable spacing, such that all the bands contain the same number of genes in it. Segmentation or grouping, respectively, is preferably achieved by providing thresholds $T_n$ that are parallel to the X axis and which divide the data into groups $G_n$. Preferably, all value pairs of a group $G_n$ satisfy $T_n < \sqrt{(X^2n+Y^2n)} < T_{(n+1)}$, before rotation, or equivalently $T_n < (Y_n') < T_{(n+1)}$ after rotation. The number of genes within the bands has to be large enough to ensure reasonable estimation of the distribution functions within these bands (preferably more then 10, more preferably more then 100, and most preferably more than 1000). Also preferably, about 20-30, and more preferably about 50-100 lines, i.e., groups, are used. Alternative ways of grouping are encompassed by the present invention which result in a defined number of groups.

In a subsequent step, the distribution functions and their characteristics such as e.g. the standard deviation and eventually higher moments and functions thereof are calculated. The distribution function f(X) is computed by means of histograms of all genes within one group after rotation. (Histograms can be smoothed or non-smoothed, can be approximated by a Gaussian distribution or taken as raw data. The binning for obtaining the histograms is a free parameter to the procedure and has to be chosen appropriately, i.e. consistent with the normalization scheme used for the gene expression data, and such that the number of populated bins is neither to low, nor to high and noisy). The characteristic moments are then computed either directly by assuming a Gaussian distribution (e.g. mean, standard deviation, skewness, or kurtosis) or by computing the moments with: $moment_1 = \int(f(X)X\,dX)$, $moment_2 = \int(f(X)X^2\,dX)$, $moment_3 = \int(f(X)X^3\,dX)$, etc. for each group separately. This is done by taking all values in a group and calculating these statistical values, preferably the standard deviation, or second moment for that group. As mentioned above, groups are chosen such that there will be sufficiently many items for reliable computation of these statistical characteristics.

The standard deviation for N samples (genes) in a group is preferably calculated with the following algorithm. $X_i'$ are the values after rotation:

$$Std(X')^2 = \frac{1}{(N-1)} \bullet \sum_{i=1}^{N}\left(X'_i - mean(X'_i)\right)^2$$

These segmentally calculated characteristics, e.g. standard deviations or other statistical measures are now used as the basis for the delineation of insignificant changes versus significant changes in gene expression. If desired, the standard deviation or other measures for each group may be plotted along the X axis of the graph, resulting in an approximately vase-shaped contour around the Y axis. This is because the distribution functions of genes with lower expression levels will be wider than that of genes with higher expression levels so that e.g. the standard deviation curve in the lower part of the graph will the broader than in the upper part of the graph.

Optionally, the statistical characteristics e.g. the standard deviation curve may be smoothed. This may be of advantage because by segmenting the data, fewer data are available for each segment to calculate the standard deviation. It is therefore possible that the number of data for some segments is too small to correctly calculate the standard deviation. This may be overcome by smoothing e.g. the standard deviation curve. A number of methods, such as calculation of the standard deviation by using an adjusted mean of n adjacent standard deviation values could be used.

Further optionally, the so obtained statistical characteristics for the individual groups such as e.g. the standard deviation curve may be rotated back by a 45° angle to provide the usual gene expression profiling result graph, but with the improved standard deviation curve surrounding the central line.

By using the improved method of calculating standard deviations, the detection of significantly changed genes is now made much easier, as false negative identifications (in cases of larger expression levels or in the upper right corner of the graph, respectively) are avoided, as well as false positive identifications (in the lower left corner of the graph or for genes with lower expression levels, respectively).

**Brief description of the Figures**

Fig. 1      shows a X-Y plot representation of expression levels in a gene profiling experiment;

Fig. 2      shows the direction of rotation of the expression values according to a preferred embodiment of the present invention;

Fig. 3      shows the data set of Fig. 1 after rotation by 45° for the embodiment of Fig. 2;

Fig. 4      shows how segmentation is achieved by providing thresholds $T_n$ that are parallel to the X axis and which divide the data into a plurality of groups $G_n$;

Fig. 5    is a flow diagram of a preferred embodiment of the present invention;
Fig. 6    shows the BEC signal intensity vs. LEC signal intensity for a larger number of genes;
Fig. 7    shows the mRNA abundance vs. the BEC/LEC ratio; for the example of Fig. 6;
Fig. 8    shows the relative mRNA abundance vs. the BEC/LEC ratio after forming equi-populated groups; for the example of Fig. 6;
Fig. 9    shows the BEC/LEC ratio vs. the relative mRNA abundance for the example of Fig. 6, however, in comparison to Fig. 8, different regions for the standard deviation are shown;
Fig. 10   shows the BEC signal intensity vs. LEC signal intensity for the example of Fig. 8 with standard deviation regions indicated; and
Fig. 11   shows the result of the standard t-test applied on the data set of Fig. 6.

**Detailed description of the invention**

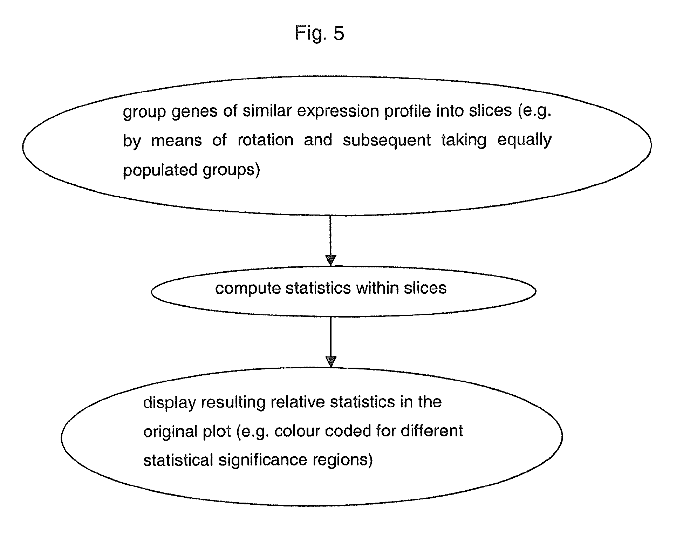

Fig. 5 shows a flow diagram of the preferred embodiment of the present invention. According to the first step shown in Fig. 5, genes of similar expression profiles are grouped into slices. As described above, this is preferably done by rotation of the data and subsequent taking equally populated groups. In the next step, the statistics within slices are calculated, as described above. Finally, the resulting relative statistics are displayed in the original plot. Preferably, different statistical significance regions are color coded.

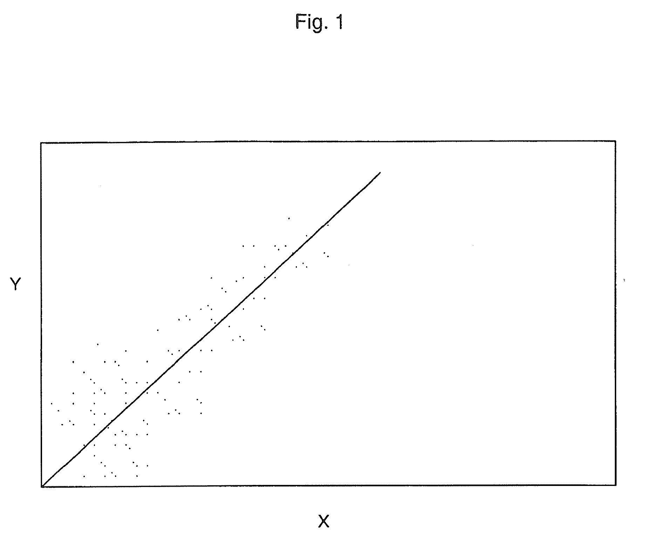

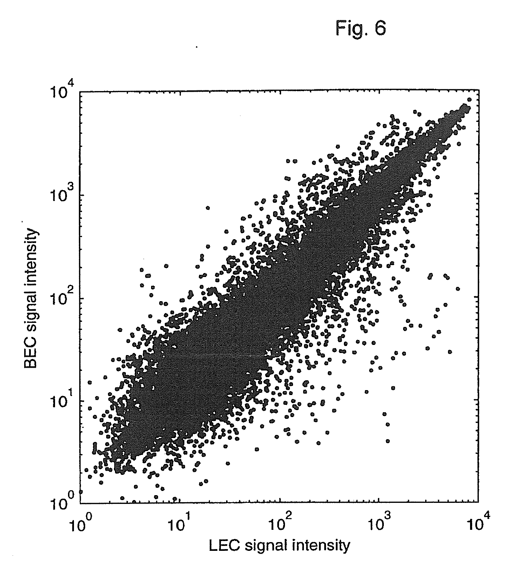

Fig. 1 shows an X-Y plot representation of expression levels in a gene profiling experiment. The X axis denotes expression values e.g., derived from lymphatic endothelial cells (LEC), whereas the Y axis denotes expression values e.g., derived from blood endothelial cells (BEC). Each gene is marked as a dot on the graph, with the position of the dot determined by the expression level of the gene. The straight line at 45 degrees denotes the locations of genes that are expressed equally in both cell lines, i.e., where there is no difference in expression level. Fig. 6 relates to the same example, showing BEC signal intensity vs. LEC signal intensity for a larger number of genes.

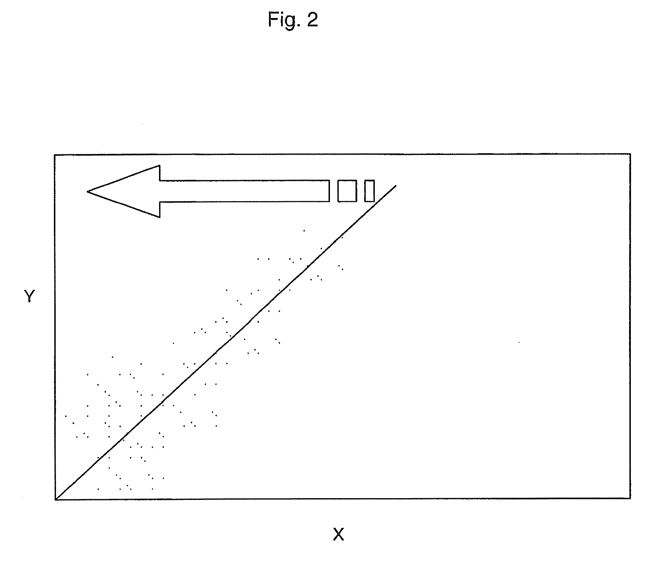

Fig. 2 shows the direction of rotation of the expression values. By rotating the data set according to a preferred embodiment of the present invention counterclockwise by 45°, the line denoting equal expression of genes in both cell lines now coincides with the Y axis of the graph.

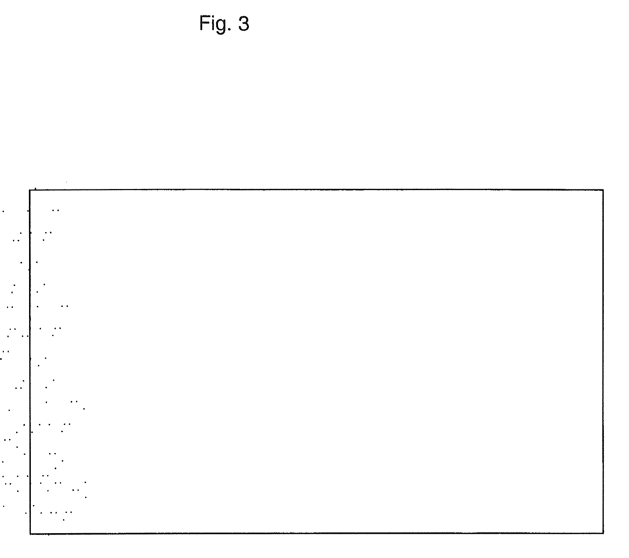

Fig. 3 shows the data set after rotation by 45°. The data are now centered around the Y axis.

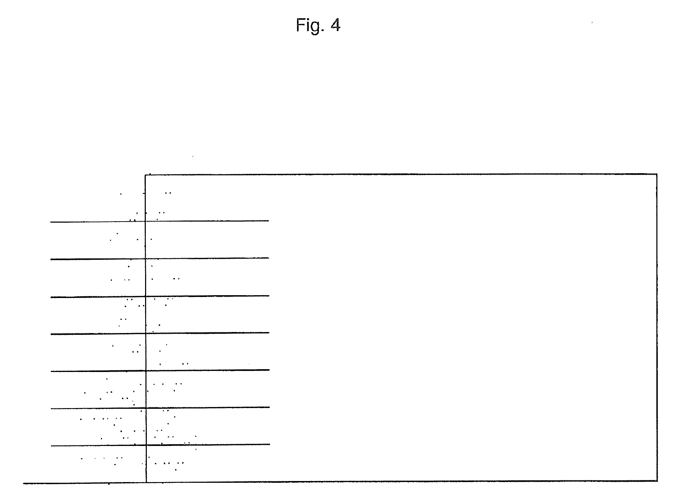

Fig. 4 shows how segmentation is achieved by providing thresholds $T_n$ that are parallel to the X axis and which divide the data into groups $G_n$. According to the preferred embodiment shown in Fig. 4, the thresholds are equally spaced from each other. On the basis of such grouped data, the significance values are calculated. After calculation of significance values, the segmented data are preferably be rotated back.

Optionally, values that are located outside of a specified significance threshold, e.g., $2\sigma$, are marked in the graph, e.g. by different (e.g., red) coloring, to enable easy identification.

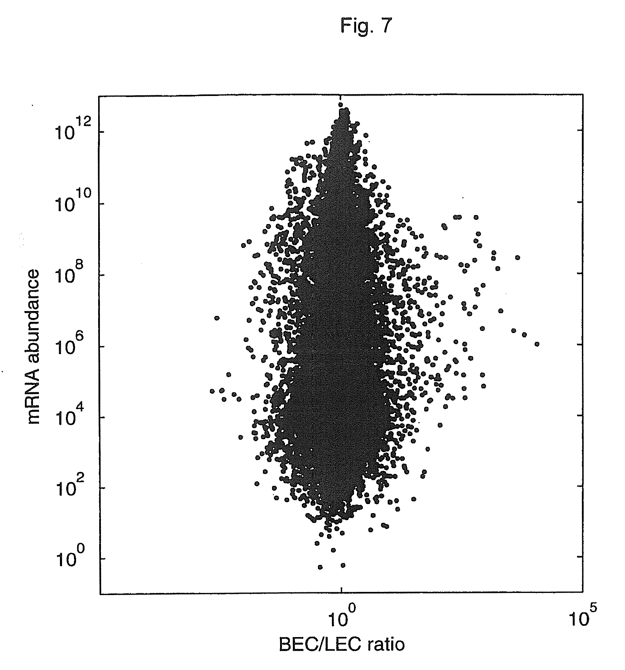

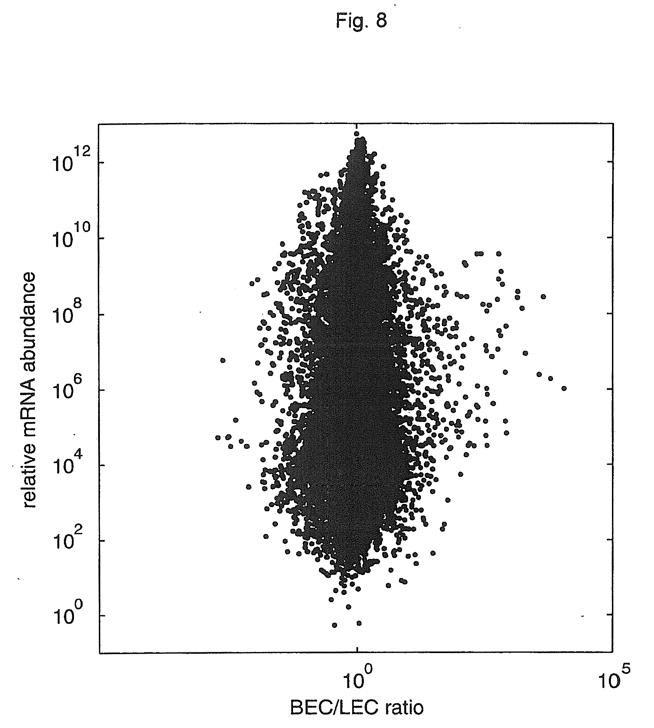

Figs 5 to 9 show a more specific example on to which the method of the present invention was applied. As mentioned above, Fig. 6 shows BEC signal intensity vs. LEC signal intensity for a larger number of genes. For the X and Y axis, the double logarithmic form is used. For this example, Fig. 7 shows the mRNA abundance vs. the BEC/LEC ratio. Furthermore, Fig. 8 shows the relative mRNA abundance vs. the BEC/LEC ratio; for the embodiment of Fig. 6.

As mentioned above, segmentally calculated characteristics, e.g. standard deviations or other statistical measures are used as the basis for the delineation of insignificant changes versus significant changes in gene expression. The standard deviation or other measures for each group are preferably plotted along the X axis of the graph, resulting in an approximately vase-shaped contour around the Y axis.

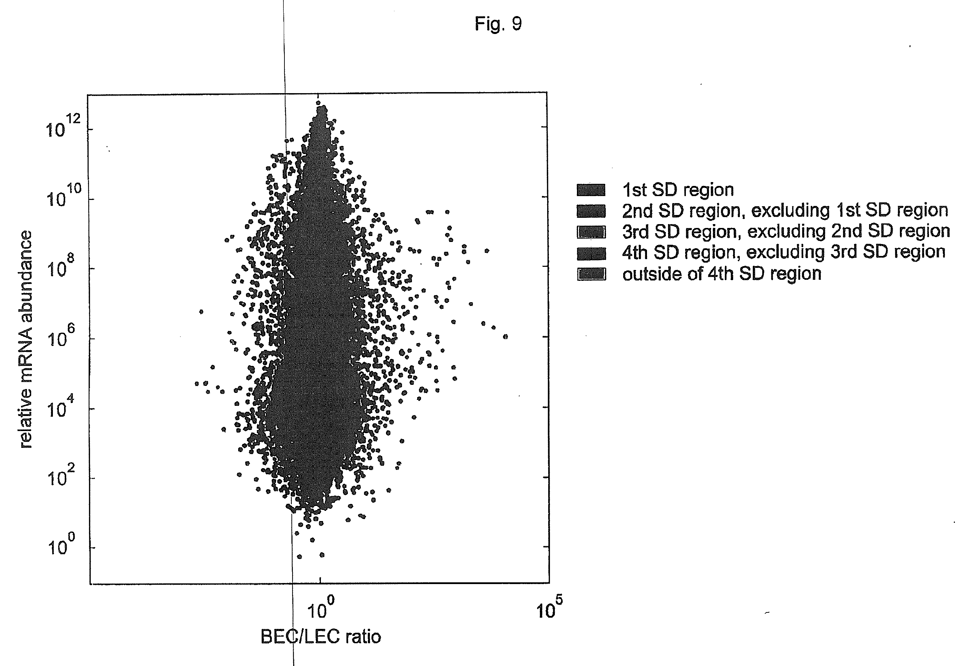

Fig. 9 also shows the BEC/LEC ratio vs. the relative mRNA abundance for the example of Fig. 6. However, in comparison to Fig. 8, different regions for the standard deviation are shown. The central region at a BEC/LEC ratio of about 1 is the first standard deviation (in the Figures "SD") region. With decreasing and increasing BEC/LEC ratios, the following regions follow in this order: second standard deviation region (excluding the first SD region), third standard deviation region (excluding the second SD region), fourth standard deviation region (excluding the third SD region), and finally the remaining data outside the fourth SD region. In order to easily visualize the different regions, the regions are preferably colored differently (in Fig. 9, different grey levels are used to differentiate the various regions).

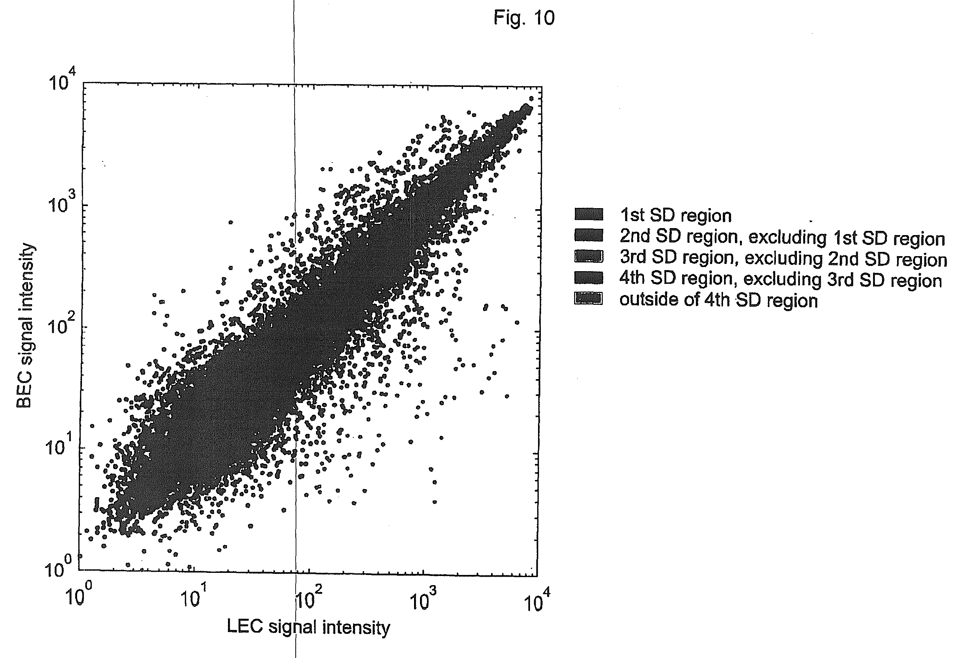

Fig. 10 shows the data after the step of rotating the data back by 45°. Again, the different standard deviation regions are shown with different colors.

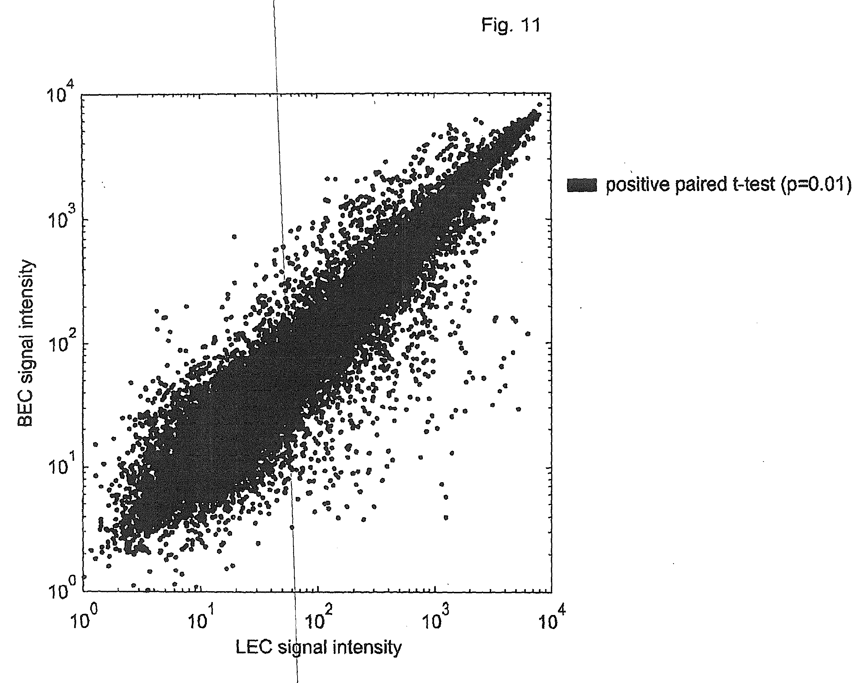

In Fig. 11, the standard prior art approach (so-called t-test) is shown. The lighter colored data plots in this Figure represent the positive paired t-test data for $p < 0.01$. A comparison of Figs. 10 and 11 clearly shows that different genes are identified.

**Claims**

1. Method for calculating statistical characteristics for a set of gene expression value pairs $(X_n | Y_n)$, comprising the steps of:

   a) grouping said set of value pairs into a plurality of groups $G_n$; and
   b) calculating the statistical characteristics for each group.

2. The method according to claim 1, wherein step a) comprises the steps of:

   a1) rotating each value pair in a X-Y-representation by a predetermined rotation angle;
   a2) dividing the range within which the value pairs are located into bands.

3. The method of claim 2, wherein in step a2) the bands are equally spaced.

4. The method according to claim 2 or 3, wherein the groups are defined by setting threshold values $T_n$ wherein all value pairs of a group $G_n$ satisfy $T_n < \sqrt{(X^2_n + V^2_n)} < T_{(n+1)}$, before rotation, or equivalently $T_n < (Y_n') < T_{(n+1)}$ after rotation.

5. The method according to any of claims 1 to 4, wherein the number of groups is about 20-30, more preferably about 50-100.

6. The method according to any of claims 1 to 5, wherein in step b) the statistical characteristics for a group is calculated by calculating the mean of the statistical characteristic of the group $G_n$.

7. The method according to claim 6 wherein the mean is a weighted mean.

8. Method of any of claims 1 to 7, wherein said statistical characteristics are standard deviation values.

9. The method according to any of claims 1 to 8, wherein said value pairs are grouped according to their expression level.

10. The method of claim 8 or 9, further comprising the step of determining whether a given pair of expression values is located within or without a predetermined significance threshold.

11. The method of claim 10, further comprising delineation of insignificant changes versus significant changes in gene expression on the basis of said segmentally calculated statistical characteristics.

Fig. 1

Fig. 2

Fig. 3

Fig. 4

EP 1 744 261 A1

Fig. 5

group genes of similar expression profile into slices (e.g. by means of rotation and subsequent taking equally populated groups)

↓

compute statistics within slices

↓

display resulting relative statistics in the original plot (e.g. colour coded for different statistical significance regions)

Fig. 6

Fig. 7

Fig. 8

Fig. 9

1st SD region
2nd SD region, excluding 1st SD region
3rd SD region, excluding 2nd SD region
4th SD region, excluding 3rd SD region
outside of 4th SD region

EP 1 744 261 A1

Fig. 10

Fig. 11

positive paired t-test (p=0.01)

EP 1 744 261 A1

**European Patent Office**

**EUROPEAN SEARCH REPORT**

Application Number

EP 05 01 5085

## DOCUMENTS CONSIDERED TO BE RELEVANT

| Category | Citation of document with indication, where appropriate, of relevant passages | Relevant to claim | CLASSIFICATION OF THE APPLICATION (Int.Cl.7) |
|---|---|---|---|
| X | WO 01/84139 A (THE BOARD OF TRUSTEES OF THE LELAND STANFORD JUNIOR UNIVERSITY) 8 November 2001 (2001-11-08)<br>* abstract *<br>* page 3, line 28 - page 5, line 6 *<br>* page 10, line 9 - page 14, line 20 *<br>* page 20, line 31 - page 21, line 15 *<br>* claim 1 *<br>----- | 1-11 | G06F19/00 |
| A | PAN WEI ET AL: "A mixture model approach to detecting differentially expressed genes with microarray data."<br>FUNCTIONAL & INTEGRATIVE GENOMICS. JUL 2003,<br>vol. 3, no. 3, July 2003 (2003-07), pages 117-124, XP002350990<br>ISSN: 1438-793X<br>* abstract *<br>* page 119, right-hand column, last paragraph - page 122, right-hand column, last paragraph *<br>----- | 1-11 | |
| A | YAN X ET AL: "Detecting differentially expressed genes by relative entropy"<br>JOURNAL OF THEORETICAL BIOLOGY, ACADEMIC PRESS, LONDON, GB,<br>vol. 234, no. 3, 7 June 2005 (2005-06-07), pages 395-402, XP004797694<br>ISSN: 0022-5193<br>* abstract *<br>* page 395, left-hand column, paragraph 1 - page 396, right-hand column, paragraph 1 *<br>* page 397, left-hand column, paragraph 2 - page 399, right-hand column, last paragraph *<br>-----<br>-/-- | 1-11 | G06F |

| | TECHNICAL FIELDS SEARCHED (Int.Cl.7) |
|---|---|
| | G06F |

The present search report has been drawn up for all claims

| Place of search | Date of completion of the search | Examiner |
|---|---|---|
| Munich | 26 October 2005 | Hilbig, M |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

**European Patent Office**

## EUROPEAN SEARCH REPORT

Application Number

EP 05 01 5085

### DOCUMENTS CONSIDERED TO BE RELEVANT

| Category | Citation of document with indication, where appropriate, of relevant passages | Relevant to claim | CLASSIFICATION OF THE APPLICATION (Int.Cl.7) |
|---|---|---|---|
| A | PAN WEI: "A comparative review of statistical methods for discovering differentially expressed genes in replicated microarray experiments" BIOINFORMATICS (OXFORD), vol. 18, no. 4, April 2002 (2002-04), pages 546-554, XP002350991 ISSN: 1367-4803 * abstract * * page 546, left-hand column, paragraph 1 - page 552, right-hand column, paragraph 1 * | 1-11 | |

-----

TECHNICAL FIELDS SEARCHED (Int.Cl.7)

The present search report has been drawn up for all claims

| Place of search | Date of completion of the search | Examiner |
|---|---|---|
| Munich | 26 October 2005 | Hilbig, M |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons
............................................................
& : member of the same patent family, corresponding document

EPO FORM 1503 03.82 (P04C01)

## ANNEX TO THE EUROPEAN SEARCH REPORT
## ON EUROPEAN PATENT APPLICATION NO.

EP 05 01 5085

This annex lists the patent family members relating to the patent documents cited in the above-mentioned European search report.
The members are as contained in the European Patent Office EDP file on
The European Patent Office is in no way liable for these particulars which are merely given for the purpose of information.

26-10-2005

| Patent document cited in search report | | Publication date | Patent family member(s) | | Publication date |
|---|---|---|---|---|---|
| WO 0184139 | A | 08-11-2001 | US | 2002019704 A1 | 14-02-2002 |

EPO FORM P0459

For more details about this annex : see Official Journal of the European Patent Office, No. 12/82